(19) Europäisches Patentamt

European Patent Office

Office européen des brevets

(11) **EP 1 320 323 B1**

(12) **EUROPÄISCHE PATENTSCHRIFT**

(45) Veröffentlichungstag und Bekanntmachung des Hinweises auf die Patenterteilung:
**15.11.2006 Patentblatt 2006/46**

(21) Anmeldenummer: **01985667.3**

(22) Anmeldetag: **25.09.2001**

(51) Int Cl.:
*A61B 5/0215* (2006.01)        *A61B 5/02* (2006.01)
*A61B 5/021* (2006.01)        *G06F 17/00* (2006.01)
*G01L 27/00* (2006.01)

(86) Internationale Anmeldenummer:
**PCT/DE2001/003719**

(87) Internationale Veröffentlichungsnummer:
**WO 2002/026127 (04.04.2002 Gazette 2002/14)**

(54) **VERFAHREN ZUR ERMITTLUNG, ÜBERWACHUNG UND AKTUALISIERUNG VON KORREKTURDATEN FÜR FLÜSSIGKEITSGEFÜLLTE ÜBERTRAGUNGSSYSTEME**

METHOD FOR ACQUIRING, MONITORING AND UPDATING CORRECTION DATA FOR LIQUID-FILLED TRANSMISSION SYSTEMS

PROCEDE POUR DETERMINER, SURVEILLER ET ACTUALISER DES DONNEES DE CORRECTION POUR DES SYSTEMES DE TRANSMISSION REMPLIS DE LIQUIDE

(84) Benannte Vertragsstaaten:
**AT BE CH CY DE DK ES FI FR GB GR IE IT LI LU MC NL PT SE TR**

(30) Priorität: **28.09.2000 DE 10049734**

(43) Veröffentlichungstag der Anmeldung:
**25.06.2003 Patentblatt 2003/26**

(73) Patentinhaber:
• **Deutsches Herzzentrum Berlin**
  **13353 Berlin (DE)**
• **Medserv GmbH**
  **04155 Leipzig (DE)**

(72) Erfinder: **WELLNHOFER, Ernst**
**10589 Berlin (DE)**

(74) Vertreter: **Ninnemann, Detlef**
**Maikowski & Ninnemann,**
**Postfach 15 09 20**
**10671 Berlin (DE)**

(56) Entgegenhaltungen:
**DE-A- 19 820 844        US-A- 5 827 195**

## Beschreibung

Die Erfindung bezieht sich auf ein Verfahren zur Ermittlung, Überwachung und Aktualisierung von Korrekturdaten zur Korrektur von Meßwertverfälschungen und zur Kalibration von flüssigkeitsgefüllten Übertragungssystemen gemäß dem Oberbegriff des Anspruchs 1.

Zur invasiven intraarteriellen und intravenösen Druckmessung werden in der Kardiologie, Intensivmedizin und Anästhesie flüssigkeitsgefüllte Übertragungssysteme eingesetzt, bei denen die Druckmessung im Körper eines Patienten erfolgt und über das als Katheter ausgebildete flüssigkeitsgefüllte Übertragungssystem zu einem außerhalb des Patientenkörpers angeordneten Druckwandler übertragen wird. In Abhängigkeit von der Länge, dem Querschnitt, dem Aufbau, den elastischen Materialeigenschaften des Katheters sowie der Zusammensetzung der im Katheter befindlichen Flüssigkeit kommt es zu unterschiedlichen Resonanzen, Dämpfungen und Energieverlusten des am körperinneren Ende des Katheters als Patientensignal erfaßten Druckmeßwertes und führen zu erheblichen Verfälschungen des Patientensignals durch das flüssigkeitgefüllte Übertragungssystem. Diese Verfälschungen lassen keine quantitative Analyse der Patientensignale zu und beeinträchtigen die qualitative Interpretation in der Diagnose und Überwachung.

Zur Vermeidung von Verfälschungen der Patientensignale durch das flüssigkeitsgefüllte Übertragungssystem wurden als Tipdrucksensoren bezeichnete Druckmeßwandler an der Spitze des als flüssigkeitsgefülltes Übertragungssystem dienenden Katheters angeordnet und das erfaßte Patientensignal in ein elektrisches Signal umgewandelt und über eine elektrische Leitung aus dem Körper des Patienten herausgeführt. Derartige Tipdrucksensoren sind jedoch sehr teuer und bezüglich ihrer Form und Größe nur in begrenztem Umfange verfügbar.

Aus der DE 1 982 208 844 A 1 ist ein Verfahren zur rechnerischen Korrektur von Meßwertverfälschungen durch das flüssigkeitgefüllte Übertragungssystem bei der Übertragung eines im Körperinneren eines Patienten gemessenen Patientensignals bekannt, bei dem das vom externen Druckwandler abgegebene elektrische Signal des (verfälschten) Patientensignals durch einen Analog-Digital-Wandler geleitet und das abgegebene digitalisierte Signal in einer Signalanalyse- und Bearbeitungseinheit, die auf der Basis einer digitalen Fourieranalyse arbeitet und eine schlagweise Analyse des digitalisierten Signals durchführt, analysiert wird. Das analysierte Signal wird dann mit empirisch ermittelten Korrekturdaten verknüpft, die aus einer Korrekturdatensatzmatrix oder als Korrekturdatensatzvektor abgerufen und als Fourierkoeffizienten abgegeben werden. Das von der Signalanalyse- und Verarbeitungseinheit korrigierte Signal wird schließlich zu einer Ausgabe- und/oder Auswerteinheit geleitet.

Der für dieses bekannte Verfahren benötigte Korrekturdatensatz kann unter anderem aus einer Referenzdruckmessung ermittelt werden. Zur Bestimmung der Übertragungsfunktion kann dabei an Stelle des unbekannten Meßsignals ein künstlich erzeugtes, bekanntes Testdrucksignal als Referenzsignal eingespeist werden, aus dessen Verfälschung dann auf die Eigenschaften des Übertragungssystems geschlossen wird.

Eine Möglichkeit besteht darin, einen Kalibrationsdatensatz aus einem an der Katheterspitze angeordneten Kalibrator zu gewinnen, was jedoch das Einhalten steriler Bedingungen erforderlich macht, eine umständliche Handhabung bedingt und Änderungen der Übertragungsfunktion berücksichtigen muß, weil beispielsweise der zum Druckmeßort und Kalibrator geführte Katheter gespült wird, Medikamente über den Katheter verabreicht werden und sogenannte "Microbubbles" die Übertragungsfunktion verändern. Treten solche Änderungen auf, ist ein Nachkalibrieren erforderlich, was aber bei liegendem, das heißt bei einem im Körper eines Patienten befindlichen Katheter nicht ohne und Kalibrator geführte Katheter gespült wird, Medikamente über den Katheter verabreicht werden und sogenannte "Microbubbles" die Übertragungsfunktion verändern. Treten solche Änderungen auf, ist ein Nachkalibrieren erforderlich, was aber bei liegendem, das heißt bei einem im Körper eines Patienten befindlichen Katheter nicht ohne weiteres durchgeführt werden kann.

Eine weitere Möglichkeit besteht darin, einen Korrekturdatensatz aus der Übertragungsfunktion mittels eines externen Kalibrationssignals zu gewinnen. Zu diesem Zweck wird auf der vom Meßort abgewandten Seite des Katheters, das heißt außerhalb des Körpers des Patienten ein Kalibrationssignal in Form eines Sprungsignals, eines Druckstoßes oder eines Rauschens abgegeben und die dabei erzeugte Eigenschwingung zur Berechnung des Korrekturdatensatzes verwendet. Bei dieser Art der Ermittlung der Übertragungsfunktion mittels eines externen Kalibrationssignals treten jedoch mehrere Probleme auf. Zum einen muß das Kalibrationssignal, das heißt das Sprungsignal, der Druckstoß oder das Rauschen sehr exakt und reproduzierbar erzeugt werden, was ein sehr sorgfältiges Arbeiten erfordert und wegen des nicht konstanten Meßverhaltens des Übertragungssystems Messungen über einen längeren Zeitraum notwendig macht und daher wegen des hohen Zeit- und Schulungsaufwandes beispielsweise in einem Krankenhaus nicht realisierbar ist. Zum anderen ist die Verwendung einer automatischen, mechanischen Vorrichtung, die am Druckwandler montiert wird, sehr teuer und erfordert darüber hinaus als mechanisches Präzisionsteil besonderen Bedienungsaufwand bei der Einrichtung und Wartung.

Bei einer Kalibration bei liegendem Katheter kommt zusätzlich das Problem der Signaltrennung hinzu, weil ansonsten die gemessene Sprungantwort auf ein Sprungsignal durch das eigentliche Patientensignal, beispielsweise ein Blutdrucksignal, zu stark verfälscht wird und damit falsche Korrekturdatensätze ermittelt werden. Die sich daraus ergebene Abhängigkeit vom Zeitpunkt der Auslösung des Sprungsignals, Stoßes auf das System oder Rauschens schränkt die Reproduzierbarkeit erheblich ein und bereitet insbesondere bei unregelmäßigen Patientensignalen einen erheblichen Aufwand.

Aus der US-A-5,827,195 ist ein Verfahren zur Verminderung des Rauschens in einem EKG-Signal bekannt, bei dem eine dem Herzschlag entsprechende Impulsfolge ausgewählt und in eine mehrdimensionale Darstellung unter Verwendung einer Kurzzeit-Fouriertransformation zur Bewertung der Zeit-Frequenz-Darstellung umgewandelt wird. Und auf die mehrdimensionale Darstellung der Impulsfolge wird eine mehrdimensionale Filterfunktion angewandt, um dadurch das Signal-Rausch-Verhältnis der Impulsfolge anzuheben. Die hierbei angewandten statistischen Ansätze zur Korrektur der EKG-Signallmpulsfolge dienen jedoch nur der Beseitigung einfacher Störungen in der Impulsfolge, die als normal verteilt angenommen werden und deren Bandbreite sich von der Bandbreite des Nutzsignals einfach trennen lässt.

Aufgabe der vorliegenden Erfindung ist es, ein Verfahren der eingangs genannten Art anzugeben, das eine Bestimmung, kontinuierliche Überwachung und laufende Aktualisierung von Kalibrationsdaten für eine Korrektur von Meßwertverfälschungen eines Patientensignafs ohne externes Kalibrationssignal ermöglicht und das wenig störanfällig gegenüber Artefakten und anderen, in den gemessenen Signalen enthaltenen Störungen ist.

Diese Aufgabe wird erfindungsgemäß durch ein Verfahren mit den Merkmalen des Anspruchs 1 gelöst.

Da die Korrekturkoeffizienten für eine Korrektur von Meßwertverfälschungen im Frequenzbereich für jede Frequenzlinie aus einer Statistik der Spektren von Signalsegmenten ermittelt werden und dabei empirisch überprüfte Annahmen über die entsprechende Statistik von unverfälschten Patientensignalen und allgemeine Eigenschaften der Übertragungsfunktion verwendet werden, die für Drucksignale charakteristisch sind, ermöglicht die erfindungsgemäße Lösung eine Bestimmung, kontinuierliche Überwachung und laufende Aktualisierung der Kalibrationsdatensätze zur Korrektur der Meßwertverfälschungen von Patientensignalen ohne ein externes Kalibrationssignal, wobei die Ermittlung, Überwachung und Aktualisierung der Korrekturdaten ausschließlich an einem späteren Zeitpunkt als die Erfassung der Daten am Körper des Patienten durchgeführt wird, und schafft ein kostengünstiges, gegenüber einer externen Kalibrationsvorrichtung wesentlich einfacher zu bedienendes und zu wartendes und gegenüber Störungen wenig anfälliges Korrekturverfahren, bei dem die Übertragungsfunktion direkt aus dem Patientensignal ermittelt wird.

Eine vorteilhafte Ausgestaltung der erfindungsgemäßen Lösung ist dadurch gekennzeichnet, dass die Korrekturdaten aus der aus dem Patientensignal gewonnenen Übertragungsfunktion ermittelt werden und dass bei der Ermittlung der Korrekturdaten empirisch überprüfte Annahmen über die entsprechende Statistik von unverfälschten Patientensignalen und allgemeinen Eigenschaften der Übertragungsfunktion verwendet werden.

Die efindungsgemäße Lösung ermöglicht folgendes Vorgehen zur Ermittlung, Überwachung und Aktualisierung der Übertragungsfunktion im Frequenzbereich, um daraus Kalibrationsdatensätze ohne externe Kalibrationsvorrichtung zu gewinnen:

Als Eingangssignal dient ein mittels handelsüblicher Kathetersysteme invasiv gemessenes Patienten(druck)signal. Eine mögliche Variante hierzu ist die Verwendung der Ableitung des Drucksignals nach der Zeit anstelle des Originalsignals.

2. Anschließend erfolgt eine Segmentierung des Signals entweder herzschlagweise oder in Form von Patientensignal-Segmenten, die hinsichtlich ihrer Länge für eine Fouriertransformation geeignet sind. Geeignet heißt dabei unter anderem, daß ab einer Mindestlänge der Vorzeichenwechsel der ersten Ableitung des Drucks nach der Zeit unter der Randbedingung eines minimalen Betrags der zweiten Ableitung des Drucks nach der Zeit gesucht wird. Eine weitere Option zur Bestimmung der geeigneten Länge besteht darin, daß ab einer Mindestlänge das Minimum des Fehlers bei Approximation des Drucksignals durch eine im höheren Frequenzbereich abgeschnittene Fourierreihe ermittelt wird. Weitere optionale Vorverarbeitungsschritte sind eine lineare Trendbereinigung und eine Vervielfachung des Segments.

3. Es folgt eine komplexe, auf die Segmentlänge abgestimmte Fouriertransformation der Segmente sowie im Falle einer Fast-Fouriertransformation eine Transformation mittels Zero-padding.

4. Der Ansatz zur Ermittlung der Übertragungsfunktion beruht auf einer segmentweisen Statistik der unter Punkt 3 ermittelten komplexen Fourierkoeffizienten. Das Signalmodell für einen komplexen Fourierkoeffizienten $S_i$ ist, da die Konvolution eine komplexe Multiplikation im Frequenzbereich darstellt:

$$S_i = t_i(m_i + v_i)$$

mit $t_i$ als multiplikativer Komponente (Konvolution mit Übertragungsfunktion), $m_i$ als Mittel und $v_i$ als variablem Anteil des aus der Patientensignalmessung ermittelten Fourierkoeffizenten $S_i$ für die Frequenzen i = 0....max. Die Anzahl n der Segmente bzw. der segmentweisen Fouriertransformationen für die Statistik wird so bestimmt, daß $(m_n - m_{n-1})^2$

$<\varepsilon$ (Mittelwertstabilität) und $\Sigma\,(s_j - m_n)^2 > \delta$ (ausreichende Varianz für Statistik) mit $m_n$ dem Mittelwert des komplexen Fourierkoeffizienten bei einer Statistik der Länge n und $s_j$ dem komplexen Wert des Fourierkoeffizienten bei einer Wiederholung j mit j=1....n sowie $\varepsilon$ und $\delta$ als gewählten Schranken. Die optimalen Schwellwerte $\varepsilon$ und $\delta$ müssen dabei empirisch bestimmt werden.

5. Folgende allgemeine Annahmen über das Patientensignal werden dabei insgesamt oder zum Teil für die Ermittlung der Übertragungsfunktion benutzt. Für alle Annahmen ist eine gute Näherung ausreichend:

    5.1. Die Übertragungsfunktion bleibt über den Zeitraum der Statistik konstant.
    5.2. Das Patientensignal enthält eine auch über kurze Zeiten variable Komponente und ist nicht streng periodisch.
    5.3. Die Übertragungsfunktion entspricht bis zur 1. Resonanz einem System 2.Ordnung
    5.4. Die Übertragungsfunktion entspricht im Frequenzbereich 1-2 Hz der Identität.
    5.5. Im Bereich sehr hoher Frequenzen mit Amplituden $<\varepsilon$ (Rauschen nahe Null) kann die Übertragungsfunktion vernachlässigt werden.

6. Die Übertragungsfunktion wird aus einer Statistik der Form

$$[\Sigma\,(s_{ij} - m_j)^p]\,/\,[\Sigma\,(s_{ij} - m_j)^q]\ \text{oder}\ (m_j)^p / (m_j)^q$$

für alle Frequenzlinien j = 1...max und für i=1...n Segmente bestimmt. Für die Parameter p und q gilt p>q. Anstelle der Mittelwerte können auch Medianwerte verwendet werden, um die Statistik gegenüber Ausreißern zu stabilisieren. Eine solche Statistik läßt sich entsprechend dem Signalmodell als eine Folge von komplexen Fourierkoeffizienten ansehen. Durch komplexe Differentiation oder Logarithmierung der entsprechenden Fourierreihe erhält man eine abgeleitete Folge von Koeffizienten mit einer gleichmäßigeren Größenverteilung über den Frequenzbereich. Im übrigen werden die Parameter p und q empirisch bestimmt. Der Sonderfall p=1 und q=0 ist zugelassen. Z.B. gilt für p=3 und q=2, daß bei unverfälschten Patientensignalen der Realteil des Logarithmus der Statistik $[\Sigma\,(s_{ij} - m_j)^3]\,/\,[\Sigma\,(s_{ij} - m_j)^2]$ für alle j linear mit dem Logarithmus der Frequenz abfällt. Eine Variante ist die zusätzliche Bestimmung von Momenten und Cumulanten höherer Ordnung zur Verbesserung des Signalmodells durch Charakterisierung der Verteilung des anregenden Signals.

7. Zur Ermittlung der Übertragungsfunktion wird das Verhältnis der tatsächlichen Statistik zu der bei einem unverfälschten Patientensignal erwarteten Statistik oder nach logarithmischer Transformation wird die ggf. gewichtete Differenz dieser Funktionen untersucht. Eine mögliche Variante zur Kombination der Fouriertransformation gemäß Punkt 3 und der logarithmischen Transformation stellt die Bestimmung des Ceptrums (homomorphe Dekonvolution) dar.

8. Zur Reduktion des Rauschens in dieser Statistik können folgende Verfahren einzeln oder kombiniert verwendet werden:

    8.1. Eine (gegebenenfalls gewichtet) gemittelte Summation mehrerer aufeinanderfolgender Ermittlungen der Übertragungsfunktion entsprechend Punkt 7.
    8.2. Eine leichte Vergröberungen der spektralen Auflösung durch Klassenbildung für mehrere Frequenzlinien,
    8.3. Eine glättende Interpolation oder Verwendung eines Median- oder Tiefpassfilters. Es kann auch ein direkte Anpassung an ein System 2. Ordnung oder eine andere glatte Funktion, beispielsweise eine Splinefunktion, mit minimaler Abweichung der aus der Statistik ermittelten verrauschten Übertragungsfunktion erfolgen.

9. Aus den Ergebnissen unter Punkt 8. lassen sich der Dämpfungskoeffizient und die Resonanzfrequenz, bzw. der Betrag der Übertragungsfunktion für die einzelnen Frequenzlinien ermitteln.
Alternativ kann die Resonanzfrequenz direkt aus einer Statistik der unter Punkt 6 aufgeführten Form ermittelt werden. Dieses Verfahren erlaubt auch eine Schätzung der Dämpfung. Außerdem korrelieren die Logarithmen der Mittelwerte, beispielsweise und noch besser die der Mediane, mit der Frequenz. Die Steigung der entsprechenden Regression bei verfälschten Patientensignalen liefert einen Schätzwert für die Dämpfung. Auch die absoluten Beträge der Mediane höherer Frequenzen sind bei stärkerer Dämpfung kleiner. Mehrere Schätzwerte für die Resonanzfrequenz und Dämpfung können bzgl. ihrer Güte gewichtet gemittelt oder mittels multipler Regressionsmodelle kombiniert werden. Entsprechende Regressionsmodelle können auch nichtlinear sein. Parameter für entsprechende Modelle lassen sich aus sytematischen in-vitro Testreihen ermitteln. Dadurch läßt sich eine sehr stabile und genaue Schätzung von Resonanzfrequenz und Dämpfung erzielen.

10. Optional kann ein iteratives Verfahren betreffend die Punkte 7-8 zur Verbesserung der Ergebnisse durchgeführt werden.

11. Die Phase kann dann aus der Übertragungsfunktion unter Annahme eines Systems 2. Ordnung eindeutig errechnet oder aus einer weiteren Statistik der unter Punkt 6 spezifizierten Form ermittelt werden, wobei die entfaltete Phase der Statistik in der Form $[\Sigma\, (s_{ij}-m_j)^3]\, /\, [\Sigma\, (s_{ij}-m_j)^2]$ bei unverfälschten Patientensignalen insbesondere eine sigmoide systematische Beziehung zur Frequenz zeigt.

12. Aus der Amplitude und der Phase der so bestimmten Übertragungsfunktion wird die komplexe Inverse dieser Funktion rechnerisch eindeutig ermittelt. Diese Inverse ist der Korrekturdatensatz für eine Korrektur im Frequenzbereich, wobei eine segmentweise Fouriertransformation der gemessenen Drucksignale entsprechend den Punkten 1-3 erfolgt.

Anhand von in der Zeichnung dargestellten Ausführungsbeispielen und Kurvenverläufen soll der der Erfindung zugrundeliegende Gedanke näher erläutert werden. Es zeigen:

Figur 1 ein Blockschaltbild einer Vorrichtung zur blinden Bestimmung der Übertragungsfunktion eines flüssigkeitsgefüllten Systems aus mit der Übertragungsfunktion konvolvierten Patientensignalen für die Ermittlung, Überwachung und Aktualisierung von Korrekturdaten zur Korrektur von Messwertverfälschungen und zur Kalibration von flüssigkeitsgefüllten Übertragungssystemen,

Figur 2 graphische Darstellungen der superponierten Spektren mehrerer Messungen des Logarithmus des Betrags der Statistik bei Messungen mit Tippdrucksensoren und bei Messungen mit durch ein flüssigkeitsgefülltes System verfälschten Messwerten,

Figur 3 eine graphische Darstellung der Spektren einer Messung des Logarithmus des Betrags der Statistik bei Messungen mit Tippdrucksensoren und bei Messungen mit durch ein flüssigkeitsgefülltes System verfälschten Messwerten mit jeweils logarithmierter Frequenzachse,

Figur 4 eine graphische Darstellung der Spektren einer Messung des Logarithmus des Betrags der Statistik, bei Messungen mit Tippdrucksensoren und bei Messungen mit durch ein flüssigkeitsgefülltes System verfälschten Messwerten mit jeweils logarithmierter Frequenzachse und mit Regressionen für den Kurvenverlauf bei der Messung mit Tippdrucksensoren und bei durch ein flüssigkeitsgefülltes System verfälschten Messwerten,

Figuren 5 und 6 eine graphische Darstellung der mittels verschiedener Methoden geglätteten Residuen von Messungen des Logarithmus des Betrags der für zwei unterschiedliche flüssigkeitsgefüllte Systeme mit per externer Kalibration unabhängig bestimmten Resonanzen und

Figur 7 eine graphische Darstellung wie in den Figuren 5 und 6 mit empirisch ermittelten Resonanzen.

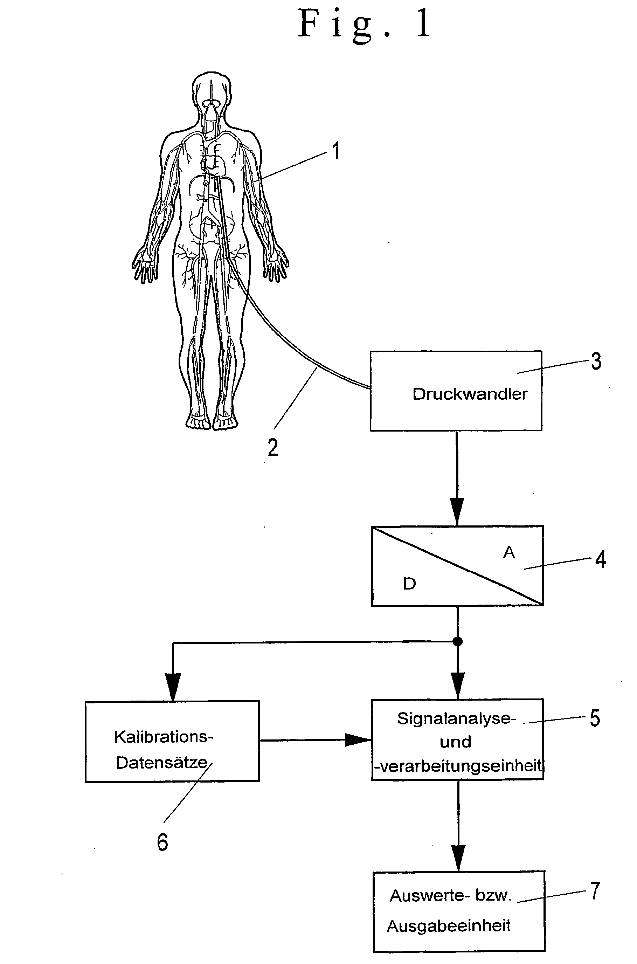

Figur 1 zeigt einen prinzipiellen Aufbau einer invasiven Druckmessung mittels eines flüssigkeitsgefüllten Übertragungssystems. Dabei wird ein das flüssigkeitsgefüllte Übertragungssystem bildender Katheter 2 durch das venöse oder arterielle System eines Patienten 1 in die Nähe der Stelle bewegt, an der beispielsweise die Impedanz am arteriellen Gefäßsystem, der Druck oder eine Ableitung des Drucks nach der zeit zur Messung der isovolumischen Kontraktionskraft oder dergleichen gemessen werden soll. Um den Patienten 1 möglichst wenig durch den Katheter 2 zu beeinflussen, weist der Katheter 2 möglichst geringe Abmessungen auf, besteht vorzugsweise aus einem elastischen Material und ist schlauchartig ausgebildet. An der Spitze des flüssigkeitsgefüllten Katheters 2 befindet sich eine Öffnung, durch die beispielsweise Druckimpulse aufgenommen und durch den Katheter 2 und eine ebenfalls flüssigkeitsgefüllte Leitung bis zu einem Druckwandler 3 weitergeleitet werden.
Der Druckwandler 3 erzeugt in Abhängigkeit von den Druckimpulsen elektrische Signale, die entsprechend dargestellt bzw. ausgewertet werden können. Dieses Verfahren ist prinzipiell seit längerem bekannt. Eine eventuelle Korrektur der Übertragungsfunktion dieses Übertragungssystems erfolgte nach Ermittlung der Resonanzfrequenz und des Dämpfungskoeffizienten mittels einer analogen elektrischen Schaltung oder eines entsprechenden numerischen Algorithmus.
Um die bei der Verwendung der oben beschriebenen Methode auftretenden Meßverfälschungen, die im Bereich von bis zu 40% liegen, wirksam zu korrigieren, wird bei dem erfindungsgemäßen Verfahren ein Analog/Digital-Wandler 4 zwischen dem Druckwandler 3 und einer Signalanalyse- und -verarbeitungseinheit 5 angeordnet, der die analogen Signale des Druckwandlers 3 in digitale, an den Eingang der Signalanalyse-und -verarbeitungseinheit 5 angelegte Signale umsetzt. Innerhalb der Signalanalyse- und -verarbeitungseinheit 5 werden die gemessenen Daten auf der Basis einer digitalen Fourieranalyse mit Korrekturfaktoren beaufschlagt und die so korrigierten Messwerte an eine Ausgabe- bzw. Auswerteeinrichtung 7 weitergeleitet.

Eine Einrichtung zur Bestimmung, kontinuierlichen Überwachung und Aktualisierung von Kalibrationsdatensätzen 6 für eine Korrektur von Messwertverfälschungen des Drucksignals ist an den Ausgang des Analog-Digitalwandlers 4 angeschlossen bzw. mit der Signalanalyse- und -verarbeitungseinheit 5 verbunden und ermittelt, überwacht und aktualisiert Parameter des Katheter-Leitungssystems und die Korrekturfaktoren.

Die digitalisierten und mit korrigierten Fourierkoeffizieten beaufschlagten Signale werden von der Signalanalyse- und -verarbeitungseinheit 5 zu einer Anzeige- bzw. Auswerteeinheit 7 übermittelt, wobei eine Anzeige sowohl auf einem Monitorsystem als auch auf einem Ausdruck erfolgen kann. Je nach Standard des Monitors, werden die Signale zunächst einem Digital/Analog-Wandler zugeführt und anschließend ausgegeben oder direkt einem Monitor überspielt, der digitale Signal verarbeiten kann. Gegebenenfalls müssen die Signale noch dergestalt aufbereitet werden, daß ein für die Darstellung geeignetes Format vorliegt.

Eine andere Möglichkeit besteht in der Übermittlung der Daten an einen Computer, der diese speichert und auswertet. In diesem Fall werden die Daten nicht in einem Digital/Analog-Wandler bearbeitet sondern werden direkt von der Korrektur weitergeleitet.

Es besteht weiterhin die Möglichkeit, die Korrektur nicht online durchzuführen, sondern die Daten abzuspeichern und zu einem späteren Zeitpunkt auszuwerten oder zu korrigieren. Voraussetzung dafür ist das Vorhandensein der systemspezifischen Daten sowie der Informationen über die Meßbedingungen, damit nachfolgend eine zutreffende Auswahl der Korrekturdatensätze erfolgen kann. Die Daten werden dafür vorteilhafterweise direkt nach dem Druckwandler 3 aufgenommen und auf einem geeigneten Speichermedium, beispielsweise einer CD oder Diskette, abgelegt.

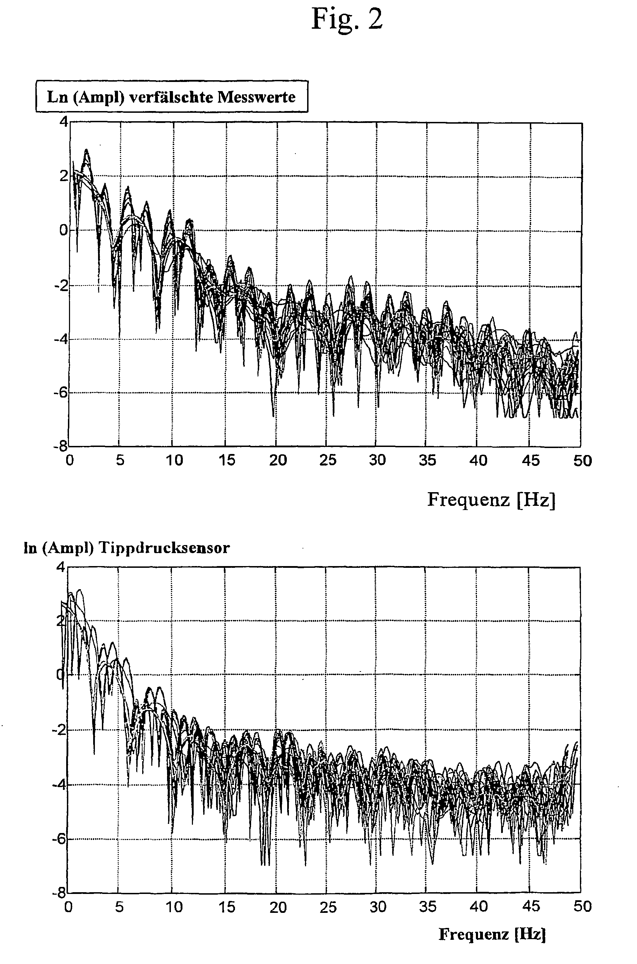

Figur 2 zeigt den Kurvenverlauf der superponierten Spektren mehrerer Messungen des Logarithmus des Betrags der Statistik (= Realteil des komlexen Logarithmus der Statistik)

$$[\sum s_{ij}-m_j)^3] \ / \ [\sum s_{ij}-m_j)^2]$$

. In der unteren Grafik ist der Kurvenverlauf für Tip-Druckkurven und in der oberen Grafik sind die durch ein flüssigkeitsgefülltes System verfälschten Messwerte dargestellt. Man erkennt deutlich den exponentiellen Abfall der Tip-Druckkurven, der bei einer Logarithmierung der Frequenzachse zu einem linearen Abfall wird. Die entsprechende Statistik der über das flüssigkeitsgefüllte Übertragungssystem verfälschten Messwerte weicht deutlich von diesem exponentiellen Abfall ab. Dieser Unterschied der Spektren der Tip-Druckkurven und der durch das flüssigkeitsgefülltes Übertragungssystem verfälschten Messwerte resultiert aus der Übertragungsfunktion.

Die Übertragungsfunktion lässt sich also ermitteln, wenn aus den in der oberen Grafik dargestellten Kurven die in der unteren Grafik dargestellten Kurven ausreichend gut geschätzt werden können. Grundsätzlich gilt, dass für alle unverzerrten Patientendruckmessungen unabhängig von der Art der Erkrankung, vom gemessenen Druck und von der individuellen Messung der exponentielle Fit sehr gut ist (r>0.9). Diese empirisch an einem umfangreichen Datensatz überprüfte Beziehung ist die Grundlage für die Schätzung des Verlaufs der Statistik für Tip-Druckkurven aus der entsprechenden Statistik für die durch das flüssigkeitsgefüllte Übertragungssystem verfälschten Messwerte.

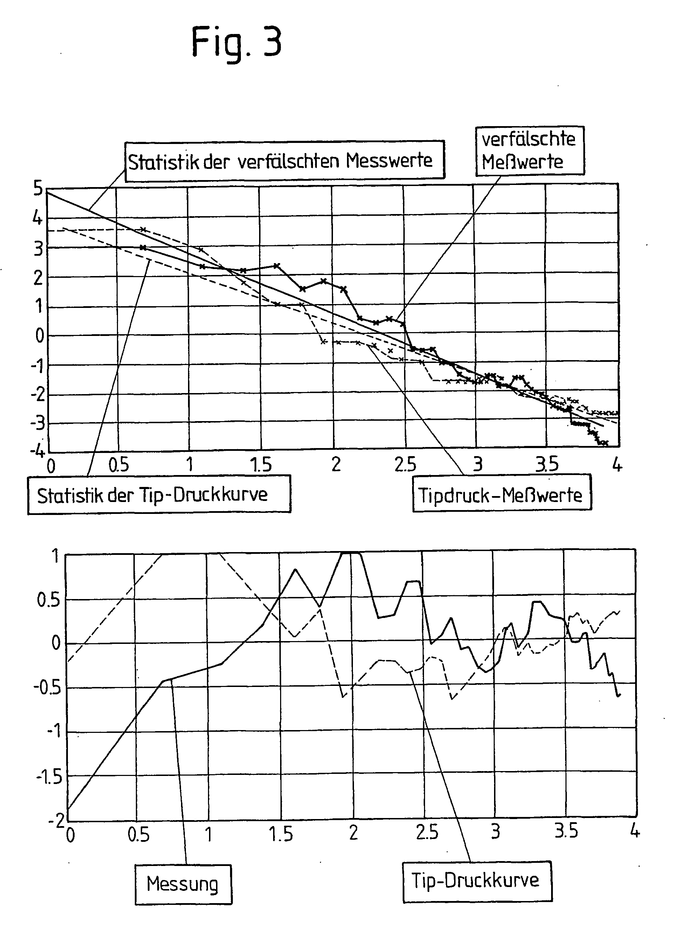

In Figur 3 sind in der oberen Darstellung die Spektren einer Messung des Logarithmus des Betrags der Statistik (= Realteil des komlexen Logarithmus der Statistik)

$$[\sum s_{ij}-m_j)^3] \ / \ [\sum s_{ij}-m_j)^2]$$

dargestellt für eine Tip-Druckkurve und eine entsprechenden Statistik für die durch das flüssigkeitsgefüllte Übertragungssystem verfälschten Messwerte. Die Frequenzachse ist logarithmiert (natürlicher Logarithmus). Man sieht deutlich die Diskrepanz im Bereich der Übertragunsfunktion.

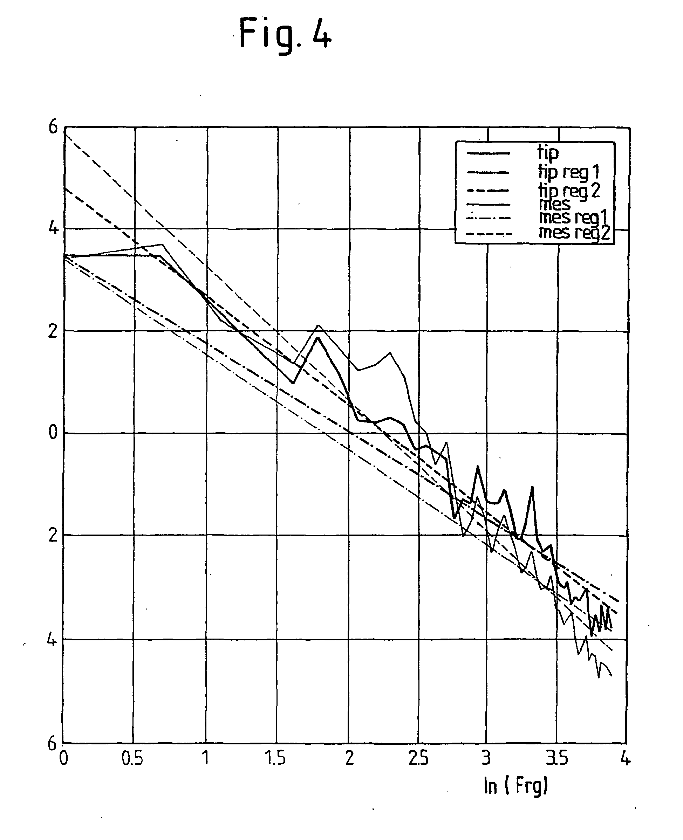

Die entsprechenden Regressionen für die Tip-Druckkurve und für die durch das flüssigkeitsgefülltes Übertragungssystem verfälschten Messwerte zeigt die untere Darstellung. Dabei lässt sich die Problematik der Ermittlung der Regression für die Tip-Druckkurven aus der Kurve der verfälschten Meßwerte verdeutlichen. Mit einer einfachen Regression ist es nicht getan, da Steigung und Offset der beiden Kurven nicht übereinstimmen. Die entsprechenden Ansätze zur Bearbeitung dieses Problems werden in Verbindung mit Figur 4 erläutert.

Bei der Analyse der Residuen nach Subtraktion der Regressionen sieht man deutlich den durch die Übertragungsfunktion verursachten Berg im Bereich der Resonanzfrequenz bei den Residuen der verfälschten Meßwerte. Die Residuen der Tip-Druckkurve oszillieren im Bereich der höheren Frequenzen um die Nulllinie, zeigen aber im Bereich der niedrigen Frequenzen einen deutlichen Berg, der durch die kräftige Grundschwingung und die ersten Oberschwingungen des Pulssignals verursacht wird und bei der Erstellung eines Signalmodells berücksichtigt werden muss.

In Figur 4 sind die Spektren einer Messung des Logarithmus des Betrags der Statistik (= Realteil des komlexen Logarithmus der Statistik)

$$[\textstyle\sum s_{ij}-m_j)^3] / [\textstyle\sum s_{ij}-m_j)^2]$$

für eine Tip-Druckkurve (tip) und eine entsprechenden Statistik für die durch das flüssigkeitsgefülltes System verfälschten Messwerte (mes) dargestellt. Die Frequenzachse ist dazu logarithmiert (natürlicher Logarithmus).

Die entsprechenden Regressionen für die Tip-Druckkurve (tip reg 1 und tip reg 2) und für die durch das flüssigkeitsgefülltes System verfälschten Messwerte (mes reg 1 und mes reg 2) sind die beiden oberen Linien, die sich in der Mitte des Bildes kreuzen. Unter Verwendung der Annahmen, dass die Übertragungsfunktion im niedrigen Frequenzbereich der Identität (Punkt 5.4. der obenstehenden Merkmalsgliederung) entspricht und ab Unterschreiten einer gewissen Signalschwelle vernachlässigbar ist (5.5. der obenstehenden Merkmalsgliederung) lässt sich eine Art Zwei-Punkt-Regression bilden (siehe die beiden unteren Geraden in Figur 4).

Zur Verfügung stehen bei Fehlen einer Tipdruck-Referenzmessung (normaler Messfall) die beiden die verfälschten Meßwerte und die Regressionen der verfälschten Meßwerte wiedergebenden Kurven. Geschätzt werden soll die die Tip-Druckmessung wiedergebende Kurve. Das gelingt mit einer gewichteten Mittelung beider Regressionen der verfälschten Messung hinreichend gut.

Diese Schätzung kann noch iterativ verbessert werden durch Grobabschätzung der Resonanzfrequenz. Auch dieser Schritt wurde empirisch in extenso überprüft.

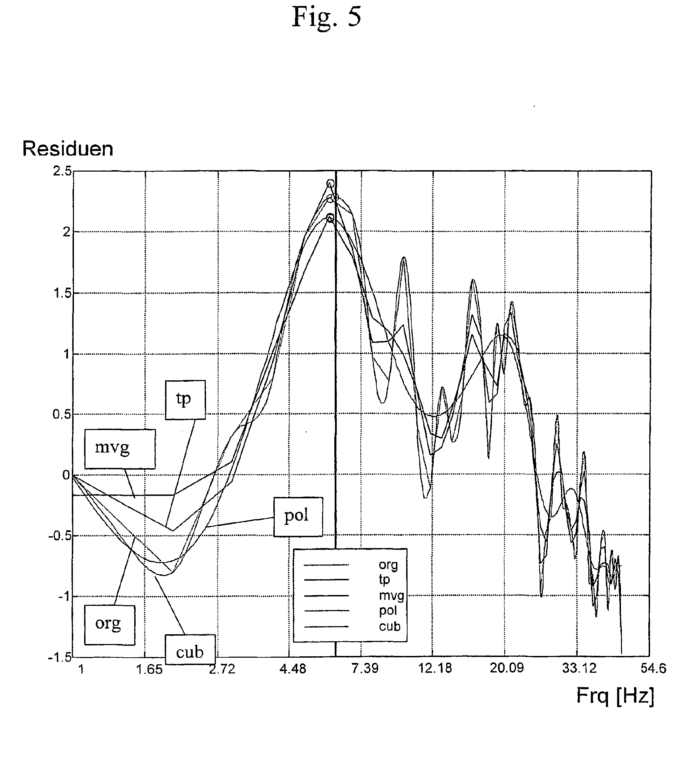

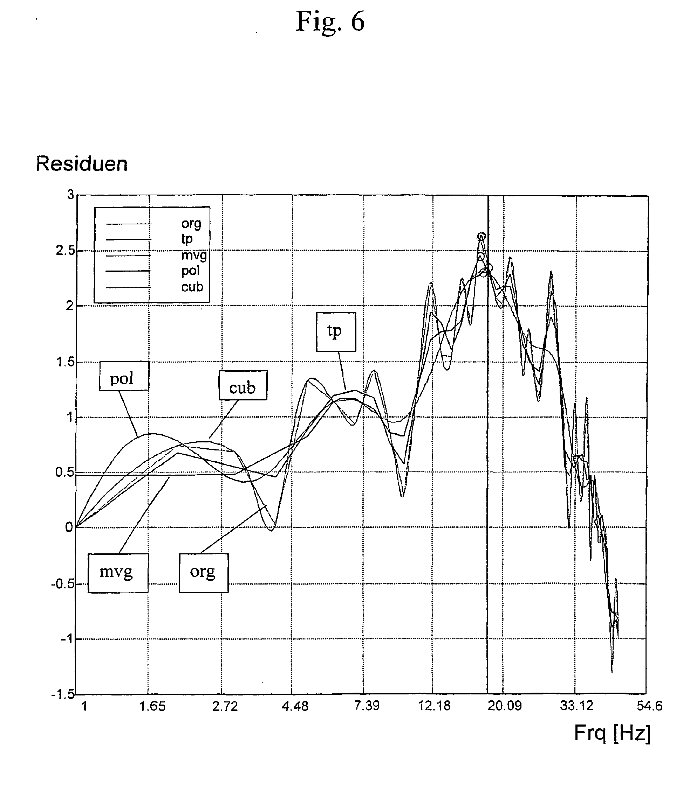

In den Figuren 5 und 6 sind die mittels verschiedener Methoden geglätteten Residuen, von Messungen des Logarithmus des Betrags der Statistik (= Realteil des komlexen Logarithmus der Statistik)

$$[\textstyle\sum s_{ij}-m_j)^3] / [\textstyle\sum s_{ij}-m_j)^2]$$

für zwei unterschiedliche flüssigkeitsgefüllte Systeme mit per externer Kalibration unabhängig bestimmten Resonanzen dargestellt.

Darin bezeichnet

"**org**" die Originalmessung, d.h. die unbearbeitete Messkurve,
"**tp**" die mittels eines Tiefpassfilters geglättete Kurve,
"**mvg**" die mittels eines Mittelwertfilters geglättete Kurve,
"**cub**" die durch Interpolation mittels eines kubischen Polynoms geglättete Kurve,
"**pol**" die mit einem allgemeinen Polynom gefilterte Kurve.

Dabei erwies sich die mittels eines Tiefpassfilters durchgeführte Glättung am stabilsten. Die empirisch ermittelten Resonanzen werden durch Ringe markiert. Die Frequenzachse ist logarithmiert, aber mit entsprechenden delogarithmierten Frequenzen beschriftet.

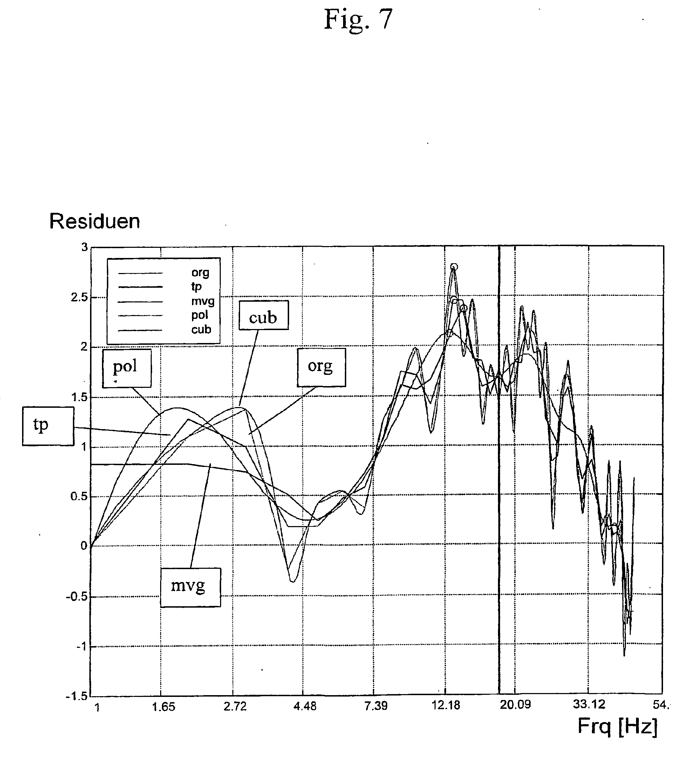

Das Ergebnis kann gemäß Figur 7, in der den Figuren 5 und 6 entsprechende Kurvenverläufe dargestellt und entsprechend bezeichnet sind, allerdings bei Messungen mit einer relativen Lücke im Spektrum des Anregungssignals (Tip-Signals, Referenzsignals) im Bereich der Resonanzfrequenz gestört werden. Ein verbessertes Interpolationsverfahren, z.B. ein Fitting an System 2. Ordnung, beseitigt dieses Problem.

Ein weiteres evtl. störendes Problem ist der Berg im Bereich der niedrigen Frequenzen des Originalsignals, der durch die kräftige Grundschwingung und die ersten Oberschwingungen des Pulssignals verursacht wird. Dieses Problem lässt sich wie folgt beheben:

1. Durch Festlegung einer Untergrenze für ein plausible Resonanzfrequenz z.B. 3 Hz.

2. Durch Nullsetzen der Residuen im niedrigen Frequenzbereich (bis 1 (2) Hz) 0.

3. Durch Verbessern des Modells dahingehend, dass der Anfang der geschätzten Referenzregression ersetzt wird durch die Messwerte mit der Annahme, dass die Übertragungsfunktion die Identität im niedrigen Frequenzbereich ist. Um Stetigkeit zu gewährleisten, bietet sich an, beide Kurven gewichtet zu mitteln, wobei das Gewicht der Messwerte bei 1Hz 1 ist und relativ rasch auf 0 abfällt, und das Gewicht der Regression bei 1 Hz 0 ist und relativ rasch auf 1 ansteigt.

Bereits ohne diese Verbesserungen (Fitting an System 2. Ordnung, und Verbesserung des Signalmodells) erlaubt das Verfahren eine exakte und von Patienten (Krankheitsbild), Art des Drucks und Übertragungssystem unabhängige Bestimmung des Dämpfungskoeffizienten und der Resonanzfrequenz, wie in systematischen Vergleichsuntersuchungen mit per externer Kalibration unabhängig bestimmter Übertragungsfunktion nachgewiesen werden konnte.

## Patentansprüche

1. Verfahren zur Ermittlung, Überwachung und Aktualisierung von Korrekturdaten zur Korrektur von durch ein flüssigkeitsgefülltes Übertragungssystem verursachte Messwertverfälschungen und zur Kalibration des flüssigkeitsgefüllten Übertragungssystems, über das im Körper eines Patienten erfasste Patientsignale zu einem externen Messwertempfänger übertragen werden, wobei die Ermittlung, Überwachung und Aktualisierung der Korrekturdaten ausschließlich an einem späteren Zeitpunkt als die Erfassung der Daten am Körper des Patienten durchgeführt wird, insbesondere für invasive Druckmessungen in der Kardiologie, Intensivmedizin und Anästhesie,
**dadurch gekennzeichnet,**
**dass** die Korrekturdaten im Frequenzbereich unter Verwendung logarithmischer Transformationen für jede Frequenz aus einer spektralen Statistik der gemessenen Patientensignale ermittelt werden.

2. Verfahren nach Anspruch 1, **dadurch gekennzeichnet, dass** die Korrekturdaten aus der aus dem Patientsignal gewonnenen Übertragungsfunktion ermittelt werden.

3. Verfahren nach Anspruch 1, **dadurch gekennzeichnet, dass** bei der Ermittlung der Korrekturdaten empirisch überprüfte Annahmen über die entsprechende Statistik von unverfälschten Patientensignalen und allgemeinen Eigenschaften der Übertragungsfunktion verwendet werden.

4. Verfahren nach Anspruch 2 oder 3, **gekennzeichnet durch**

   a) eine Segmentierung des gemessenen Patientensignals,
   b) eine komplexe, auf die Segmentlänge abgestimmte Fouriertransformation der Segmente,
   c) eine segmentweise Statistik der ermittelten komplexen Fourierkoeffizienten und
   d) Ermittlung der Übertragungsfunktion aus dem Verhältnis der tatsächlichen, segmentweisen Statistik der ermittelten, komplexen Fourierkoeffizienten zu der bei einem unverfälschten Patientensignal erwarteten Statistik der komplexen Fourierkoeffizienten.

5. Verfahren nach Anspruch 4, **dadurch gekennzeichnet, dass** die komplexe, auf die Segmentlänge abgestimmte Fouriertransformation der Segmente bei einer Fast-Fouriertransformation mittels Zero-Padding erfolgt.

6. Verfahren nach Anspruch 2 oder 3, **gekennzeichnet durch**

   a) eine Segmentierung des gemessenen Patientensignals,
   b) eine komplexe, auf die Segmentlänge abgestimmte Fouriertransformation der Segmente,
   c) eine segmentweise Statistik der ermittelten komplexen Fourierkoeffizienten,
   d) eine logarithmische Transformation der tatsächlichen, segmentweisen Statistik und eine Schätzung der entsprechenden, bei einem unverfälschten Patientensignal zu erwartenden Statistik und
   e) eine Ermittlung der Übertragungsfunktion aus der Differenz der logarithmischen Transformation der Statistiken.

7. Verfahren nach Anspruch 2 oder 3, **gekennzeichnet durch**

   a) eine Segmentierung des gemessenen Patientensignals,
   b) eine komplexe, auf die Segmentlänge abgestimmte Fouriertransformation der Segmente,
   c) eine segmentweise Statistik der ermittelten komplexen Fourierkoeffizienten,

d) eine Differentiation der aus den Werten der Statistik als Koeffizienten gebildeten Fourierreihe und

e) eine Ermittlung der Resonanz und Dämpfung aus den Koeffizienten der differenzierten Reihe.

**8.** Verfahren nach Anspruch 2 oder 3, **gekennzeichnet durch** eine Segmentierung des gemessenen Patientensignals und Bestimmung des Ceptrums.

**9.** Verfahren nach mindestens einem der voranstehenden Ansprüche, **dadurch gekennzeichnet, dass** die Segmentierung eines invasiv mittels eines Kathetersystems gemessenen Patientendrucksignals zur invasiven intraarteriellen und/oder intravenösen Druckmessung herzschlagweise erfolgt.

**10.** Verfahren nach mindestens einem der voranstehenden Ansprüche, **dadurch gekennzeichnet, dass** die Segmentierung in Patientensignalsegmenten erfolgt, die hinsichtlich ihrer Länge für eine Fouriertransformation geeignet sind.

**11.** Verfahren nach Anspruch 10, **dadurch gekennzeichnet, dass** eine für die Fouriertransformation geeignete Länge des Patientensignalsegments **dadurch** bestimmt wird, dass ab einer vorgebbaren Mindestlänge ein Vorzeichenwechsel der ersten Ableitung des gemessenen Patientensignals nach der Zeit unter der Randbedingung eines minimalen Betrags der zweiten Ableitung des Patientensignals nach der Zeit auftritt.

**12.** Verfahren nach Anspruch 10, **dadurch gekennzeichnet, dass** eine für die Fouriertransformation geeignete Länge des Patientensignalsegments dadurch bestimmt wird, dass ab einer vorgebbaren Mindestlänge ein Minimum des Fehlers bei Approximation des Patientensignals durch eine im höheren Frequenzbereich abgeschnittene Fourierreihe auftritt.

**13.** Verfahren nach mindestens einem der voranstehenden Ansprüche, **gekennzeichnet durch** eine Trendbereinigung der Segmentierung des Patientensignals bzw. Patientendrucksignals.

**14.** Verfahren nach mindestens einem der voranstehenden Ansprüche, **gekennzeichnet durch** eine Vervielfachung des segmentierten Patientensignals bzw. Patientendrucksignals.

**15.** Verfahren nach mindestens einem der voranstehenden Ansprüche, **dadurch gekennzeichnet, dass** das Signalmodell für einen komplexen Fourierkoeffizienten $S_i$

$$S_i = t_i(m_i + v_i)$$

ist, wobei $t_i$ eine multiplikative Komponente (Konvolution mit Übertragungsfunktion), $m_i$ ein Mittelwert und $v_i$ ein variabler Anteil des aus einer Messung ermittelten Fourierkoeffizenten $S_i$ für die Frequenzen $i = 0...$ maximal ist und die Anzahl n der Segmente bzw. segmentweisen Fouriertransformationen für die Statistik so bestimmt wird, daß durch die Bedingungen

$$(m_n - m_{n-1})^2 < \varepsilon$$

eine hinreichende Mittelwertstabilität und

$$\Sigma (s_j - m_n)^2 > \delta$$

eine ausreichende Varianz für die Statistik mit $m_n$ einem Mittelwert des komplexen Fourierkoeffizienten bei einer Statistik der Länge n und $s_j$ einem komplexen Wert des Fourierkoeffizienten bei einer Wiederholung j mit $j = 1....n$ sowie $\varepsilon$ und $\delta$ als gewählten Schranken gegeben ist.

**16.** Verfahren nach Anspruch 15, **dadurch gekennzeichnet, dass** die optimalen Schwellwerte $\varepsilon$ und $\delta$ empirisch bestimmt werden.

**17.** Verfahren nach Anspruch 15 oder 16, **dadurch gekennzeichnet, dass** die Übertragungsfunktion aus einer Statistik der Form $[\Sigma\,(s_{ij}-m_j)^p]\,/\,[\Sigma\,(s_{ij}-m_j)^q]$ für alle Frequenzlinien j = l...max und für i =1...n Segmente bestimmt wird, wobei für die Parameter p und q die Bedingung p>q gilt und die Parameter p und q empirisch bestimmt werden.

**18.** Verfahren nach Anspruch 15 oder 16, **dadurch gekennzeichnet, dass** Medianwerte als Schätzwerte verwendet werden, die durch komplexe Differentiation oder Logarithmierung der entsprechenden Fourierreihe und einer daraus abgeleiteten Folge von Koeffizienten, mit einer gleichmäßigen Größenverteilung über den Frequenzbereich gewonnen werden.

**19.** Verfahren nach Anspruch 17 oder 18, **dadurch gekennzeichnet, dass** zusätzlich Momenten oder Cumulanten höherer Ordnung bestimmt werden.

**20.** Verfahren nach mindestens einem der voranstehenden Ansprüche, **gekennzeichnet durch** eine Mittelung mehrerer aufeinanderfolgender Ermittlungen der Übertragungsfunktion.

**21.** Verfahren nach Anspruch 20, **gekennzeichnet durch** eine gewichtete Mittelung oder Kombination mehrerer aufeinanderfolgender Ermittlungen der Übertragungsfunktion in einem statistischen Modell.

**22.** Verfahren nach mindestens einem der voranstehenden Ansprüche, **gekennzeichnet durch** eine formale Modellierung der nacheinander ermittelten Übertragungsfunktion als Raum-Zustandsmodell.

**23.** Verfahren nach mindestens einem der voranstehenden Ansprüche, **gekennzeichnet durch** eine Vergröberungen der spektralen Auflösung **durch** Klassenbildung für mehrere Frequenzlinien.

**24.** Verfahren nach mindestens einem der voranstehenden Ansprüche, **gekennzeichnet durch** eine glättende Interpolation der ermittelten Übertragungsfunktion und/oder Filterung der Übertragungsfunktion mittels eines Tiefpassfilters.

**25.** Verfahren nach mindestens einem der voranstehenden Ansprüche, **gekennzeichnet durch** eine direkte Anpassung an ein System 2. Ordung mit minimaler Abweichung der aus der Statistik ermittelten verrauschten Übertragungsfunktion.

**26.** Verfahren nach mindestens einem der voranstehenden Ansprüche, **dadurch gekennzeichnet, dass** nach einer Reduktion des Rauschens in der aus dem Verhältnis der tatsächlichen Statistik zu der bei einem unverfälschten Patientensignal erwarteten Statistik oder der Differenz dieser Funktionen nach einer logarithmischen Transformation ermittelten Übertragungsfunktion der Dämpfungskoeffizient und die Resonanzfrequenz, bzw. der Betrag der Übertragungsfunktion für die einzelnen Frequenzlinien ermittelt werden.

**27.** Verfahren nach den voranstehenden Ansprüchen 20 bis 26, **gekennzeichnet durch** ein iteratives Verfahren zur Verbesserung der Ergebnisse.

**28.** Verfahren nach mindestens einem der voranstehenden Ansprüche, **dadurch gekennzeichnet, dass** die Phase aus der Übertragungsfunktion errechnet wird.

**29.** Verfahren nach mindestens einem der voranstehenden Ansprüche 1 bis 27, **dadurch gekennzeichnet, dass** die Phase aus einer weiteren Statistik der in den Ansprüchen 17 und 18 spezifizierten Form ermittelt wird.

**30.** Verfahren nach mindestens einem der voranstehenden Ansprüche, **dadurch gekennzeichnet, dass** aus der Amplitude und der Phase der so bestimmten Übertragungsfunktion die komplexe Inverse dieser Funktion rechnerisch ermittelt wird, die als Korrekturdatensatz für eine Korrektur im Frequenzbereich dient, wobei eine segmentweise Fouriertransformation der gemessenen Patientensignale erfolgt

**Claims**

**1.** Method for determining, monitoring and updating correction data for correcting measured value distortions caused by a liquid-filled transmission system and for calibrating the liquid-filled transmission systems, via which signals from the patient detected in the body of a patient are transmitted to an external measured value receiver, whereby the determining, monitoring and updating correction data is carried out exclusively at a later point in time after the determination of the data in the body of the patient, in particular for invasive pressure measurements in cardiology, intensive care medicine and anaesthesia,
**characterised in that**
the correction data are determined by use of logarithmic transformations in the frequency range for each frequency from a spectral statistic of the measured patient signals.

2. Method according to claim 1, **characterised in that** the correction data are determined from the transmission function obtained from the signal from the patient.

3. Method according to claim 1, **characterised in that** in determining the correction data, empirically checked assumptions about the corresponding statistics of distorted signals from the patient and general properties of the transmission function are used.

4. Method according to claim 2 or 3, **characterised by**

   a) a segmenting of the measured signal from the patient,
   b) a complex Fourier transformation of the segments matched to the segment length,
   c) segment by segment statistics of the complex Fourier coefficients determined and
   d) determining the transmission function from the ratio of the actual segment by segment statistics of the complex Fourier coefficients determined to the statistics of the complex Fourier coefficients to be expected in an undistorted signal from the patient.

5. Method according to claim 4, **characterised in that** the complex Fourier transformation of the segments matched to the segment length takes place in a fast Fourier transformation by means of zero padding.

6. Method according to claim 2 or 3, **characterised by**

   a) segmenting of the measured signal from the patient,
   b) a complex Fourier transformation of the segments matched to the segment length,
   c) segment by segment statistics of the complex Fourier coefficients determined,
   d) a logarithmic transformation of the actual, segment by segment statistics and an estimation of the corresponding statistics to be expected in the case of an undistorted signal from the patient and
   e) determination of the transmission function from the difference in the logarithmic transformation of the statistics.

7. Method according to claim 2 or 3, **characterised by**

   a) segmenting of the measured signal from the patient,
   b) a complex Fourier transformation of the segments matched to the segment length,
   c) segment by segment statistics on the complex Fourier coefficients obtained,
   d) differentiation of the Fourier series formed from the values of the statistics as coefficients and
   e) determining the resonance and damping from the coefficients of the differentiated series.

8. Method according to claim 2 or 3, **characterised by** a segmenting of the measured signal from the patient and determination of the cepstrum.

9. Method according to at least one of the preceding claims, **characterised in that** the segmenting of a pressure signal from the patient measured invasively by means of a catheter system for the invasive intra-arterial and/or intra-venal pressure measurement takes place heartbeat by heartbeat.

10. Method according to at least one of the preceding claims, **characterised in that** the segmentation takes place in signal from the patient segments which are suitable with respect to their length for a Fourier transformation.

11. Method according to claim 10, **characterised in that** a length of the signal from the patient segment suitable for the Fourier transformation is determined **in that** from a presettable minimum length, a sign change of the first derived function of the measured signal from the patient occurs according to time with the boundary condition of a minimum amount of the second derived function of the signal from the patient according to time.

12. Method according to claim 10, **characterised in that** a length of the signal segment from the patient suitable for the Fourier transformation is determined **in that** from a presettable minimum length, a minimum fault occurs in approximation of the signal from the patient by a Fourier series cut off in the higher frequency range.

13. Method according to at least one of the preceding claims, **characterised by** a trend adjustment of the segmentation of the signal from the patient or pressure signal from the patient.

14. Method according to at least one of the preceding claims, **characterised by** a multiplication of the segmented signal from the patient or pressure signal from the patient.

15. Method according to at least one of the preceding claims, **characterised in that** the signal model for a complex Fourier coefficient Si is

$$Si = ti(mi + vi),$$

wherein ti is at most a multiplicative component (convolution with transmission function), mi is a mean value and vi is a variable portion of the Fourier coefficient Si determined from a measurement for the frequencies i = 0 ... and the number n of the segments or segment by segment Fourier transformations is determined for the statistics such that by the conditions

$$(mn - mn-1)2 < \varepsilon$$

an adequate mean value stability and

$$\Sigma (sj - mn)2 > \delta$$

an adequate variance are provided for the statistics with mn a mean value of the complex Fourier coefficient in statistics of the length n and sj, a complex value of the Fourier coefficient in a repetition j with j = 1 ... n and $\varepsilon$ and $\delta$ as selected bounds.

16. Method according to claim 15, **characterised in that** the optimum threshold values $\varepsilon$ and $\delta$ are determined empirically.

17. Method according to claim 15 or 16, **characterised in that** the transmission function is determined from statistics of the form $[\Sigma(sij - mj)p]/[\Sigma(sij - mj)q]$ for all the frequency lines j = 1 ... maximum and for i = 1 ... n segments, wherein for the parameters p and q the condition p > q applies and the parameters p and q are determined empirically.

18. Method according to claim 15 or 16, **characterised in that** median values are used as estimate values which are obtained by a complex differentiation or logarithmisation of the corresponding Fourier series and a sequence of coefficients derived therefrom, with a uniform size distribution over the frequency range.

19. Method according to claim 17 or 18, **characterised in that** additional higher-order moments or cumulants are determined.

20. Method according to at least one of the preceding claims, **characterised by** an averaging of a plurality of consecutive determinations of the transmission function.

21. Method according to claim 20, **characterised by** a weighted averaging or combination of a plurality of consecutive determinations of the transmission function in a statistical model.

22. Method according to at least one of the preceding claims, **characterised by** a formal modelling of the transmission function determined consecutively as a space-state model.

23. Method according to at least one of the preceding claims, **characterised by** a coarsening of the spectral resolution by class formation for a plurality of frequency lines.

24. Method according to at least one of the preceding claims, **characterised by** a flattening interpolation of the determined transmission function and/or filtering of the transmission function by means of a low-pass filter.

25. Method according to at least one of the preceding claims, **characterised by** a direct adaptation to a second-order system with minimal deviation of the noisy transmission function determined from the statistics.

26. Method according to at least one of the preceding claims, **characterised in that** after a reduction in the noise in the statistics expected from the ratio of the actual statistics to the statistics in an undistorted signal from the patient or the difference between these functions after a logarithmic transformation of the determined transmission function, the damping coefficient and the resonance frequency or the amount of the transmission function are determined for the individual frequency lines.

27. Method according to the preceding claims 20 to 26, **characterised by** an iterative method for improving the results.

28. Method according to at least one of the preceding claims, **characterised in that** the phase is calculated from the transmission function.

29. Method according to at least one of the preceding claims 1 to 27, **characterised in that** the phase is determined from further statistics of the form specified in claims 17 and 18.

30. Method according to at least one of the preceding claims, **characterised in that** the complex inverse of this function is determined by computer from the amplitude and the phase of the transmission function determined in this way, the complex inverse serving as the correction data record for a correction in the frequency range, a segment by segment Fourier transformation of the measured signals from the patient taking place.

## Revendications

1. Procédé de détermination, surveillance et actualisation des données de correction pour corriger des altérations des valeurs de mesure occasionnées par un système de transmission contenant un liquide et pour calibrer le système de transmission contenant un liquide, par lequel des signaux du patient, enregistrés dans le corps d'un patient, sont transmis vers un récepteur de valeurs de mesure externe, la détermination, la surveillance et l'actualisation des données de correction étant effectuées exclusivement à un moment ultérieur à l'enregistrement des données sur le corps du patient, en particulier pour des mesures de pression invasives en cardiologie, médecine intensive et anesthésie,
**caractérisé en ce que** les données de correction sont déterminées dans le domaine de fréquences moyennant l'utilisation de transformations logarithmiques pour chaque fréquence à partir d'une statistique spectrale des signaux du patient mesurés.

2. Procédé selon la revendication 1, **caractérisé en ce que** les données de correction sont déterminées à partir de la fonction de transfert obtenue à partir du signal du patient.

3. Procédé selon la revendication 1, **caractérisé en ce que** lors de la détermination des données de correction sont utilisées des hypothèses, vérifiées empiriquement, sur la statistique correspondante de signaux du patient non altérés et des propriétés générales de la fonction de transfert.

4. Procédé selon la revendication 2 ou 3, **caractérisé par**

   a) une segmentation du signal du patient mesuré,
   b) une transformation de Fourier complexe des segments, ajustée à la longueur des segments,
   c) une statistique par segments des coefficients de Fourier complexes déterminés,
   d) une détermination de la fonction de transfert à partir du rapport entre la statistique par segments réelle des coefficients de Fourier complexes déterminés et la statistique des coefficients de Fourier complexes attendue avec un signal du patient non altéré.

**5.** Procédé selon la revendication 4, **caractérisé en ce que** la transformation de Fourier complexe des segments, ajustée à la longueur des segments, est effectuée lors d'une transformée de Fourier rapide au moyen du zéro padding.

**6.** Procédé selon la revendication 2 ou 3, **caractérisé par**

a) une segmentation du signal du patient mesuré,
b) une transformation de Fourier complexe des segments, ajustée à la longueur des segments,
c) une statistique par segments des coefficients de Fourier complexes déterminés,
d) une transformation logarithmique de la statistique par segments réelle et une évaluation de la statistique correspondante à attendre lors d'un signal du patient non altéré, et
e) une détermination de la fonction de transfert à partir de la différence de la transformation logarithmique des statistiques.

**7.** Procédé selon la revendication 2 ou 3, **caractérisé par**

a) une segmentation du signal du patient mesuré,
b) une transformation de Fourier complexe des segments, ajustée à la longueur des segments,
c) une statistique par segments des coefficients de Fourier complexes déterminés,
d) une différenciation de la série de Fourier formée en tant que coefficients à partir des valeurs de la statistique, et
e) une détermination de la résonance et de l'atténuation à partir des coefficients de la série différenciée.

**8.** Procédé selon la revendication 2 ou 3, **caractérisé par** une segmentation du signal du patient et une détermination du cepstre.

**9.** Procédé selon au moins l'une quelconque des revendications précédentes, **caractérisé en ce que** la segmentation d'un signal de pression du patient, mesurée de manière invasive au moyen d'un système de cathéter, est effectuée par les battements du coeur en vue d'une mesure invasive de la pression intra-artérielle et/ou intraveineuse.

**10.** Procédé selon au moins l'une quelconque des revendications précédentes, **caractérisé en ce que** la segmentation est effectuée en segments du signal du patient, qui, du point de vue de leur longueur, sont adaptés à une transformation de Fourier.

**11.** Procédé selon la revendication 10, **caractérisé en ce qu'**une longueur du segment du signal du patient, appropriée à la transformation de Fourier, est déterminée par le fait qu'à partir d'une longueur minimum prédéfinissable, un changement de signe de la première dérivée en fonction du temps du signal du patient mesuré se produit sous la condition limite d'une valeur minimum de la deuxième dérivée du signal du patient en fonction du temps.

**12.** Procédé selon la revendication 10, **caractérisé en ce qu'**une longueur du segment du signal du patient, appropriée à la transformation de Fourier, est déterminée par le fait qu'à partir d'une longueur minimum prédéfinissable, il se produit un minimum de l'erreur lors de l'approximation du signal du patient par une série de Fourier coupée dans le domaine de fréquence élevé.

**13.** Procédé selon au moins l'une quelconque des revendications précédentes, **caractérisé par** une élimination de la tendance de la segmentation du signal du patient, plus précisément du signal de pression du patient.

**14.** Procédé selon au moins l'une quelconque des revendications précédentes, **caractérisé par** une multiplication du signal du patient segmenté, plus précisément du signal de pression du patient.

**15.** Procédé selon au moins l'une quelconque des revendications précédentes, **caractérisé en ce que** le modèle de signal pour un coefficient de Fourier complexe $S_i$ est

$$S_i = t_i(m_i + v_i)$$

où $t_i$ une composante multiplicative (convolution avec fonction de transfert), $m_i$ une valeur moyenne et $v_i$ une part

variable du coefficient de Fourier $S_i$ déterminé par une mesure pour les fréquences i = 0... sont maximales et le nombre n de segments, plus précisément de transformations de Fourier par segments, pour la statistique est déterminé de telle sorte que par les conditions

$$(m_n - m_{n-1})^2 < \epsilon,$$

on obtient une stabilité de valeur moyenne suffisante et

$$\Sigma(s_j - m_n)^2 > \delta$$

une variance suffisante pour la statistique avec $m_n$, une valeur moyenne du coefficient de Fourier complexe lors d'une statistique de la longueur n, et $s_j$, une valeur complexe du coefficient de Fourier lors d'une réitération j avec j = 1 ... n, ainsi que $\epsilon$ et $\delta$ en tant que limites choisies.

16. Procédé selon la revendication 15, **caractérisé en ce que** les valeurs de seuils optimales $\epsilon$ et $\delta$ sont déterminées empiriquement.

17. Procédé selon la revendication 15 ou 16, **caractérisé en ce que** la fonction de transfert est déterminée à partir d'une statistique de la forme $[\Sigma(s_{ij} - m_j)^p] / [\Sigma(s_{ij} - m_j)^q]$ pour toutes les lignes de fréquence j = 1... max et pour i = 1 ... n segments, la condition p > q étant appliquée pour les paramètres p et q et les paramètres p et q étant déterminés empiriquement.

18. Procédé selon la revendication 15 ou 16, **caractérisé en ce que** des valeurs médianes sont utilisées comme valeurs d'évaluation, lesquelles ont été obtenues par une différenciation complexe ou un calcul logarithmique de la série de Fourier correspondante et une suite de coefficients, dérivée de celle-ci, avec une répartition régulière des grandeurs sur le domaine de fréquence.

19. Procédé selon la revendication 17 ou 18, **caractérisé en ce que** sont déterminés, en plus, des moments ou des cumulantes d'ordre supérieur.

20. Procédé selon au moins l'une quelconque des revendications précédentes, **caractérisé par** un calcul de moyenne de plusieurs déterminations successives de la fonction de transfert.

21. Procédé selon la revendication 20, **caractérisé par** un calcul de moyenne pondéré ou une combinaison de plusieurs déterminations successives de la fonction de transfert dans un modèle statistique.

22. Procédé selon au moins l'une quelconque des revendications précédentes, **caractérisé par** une modélisation formelle de la fonction de transfert déterminée successivement sous forme de modèle d'état spatial.

23. Procédé selon au moins l'une quelconque des revendications précédentes, **caractérisé par** un grossissement grossier de la résolution spectrale par une formation de classes pour plusieurs lignes de fréquence.

24. Procédé selon au moins l'une quelconque des revendications précédentes, **caractérisé par** une interpolation de lissage de la fonction de transfert déterminée et/ou un filtrage de la fonction de transfert au moyen d'un filtre passe-bas.

25. Procédé selon au moins l'une quelconque des revendications précédentes, **caractérisé par** une adaptation directe à un système de 2$^e$ ordre avec un écart minimum de la fonction de transfert bruitée, déterminée à partir de la statistique.

26. Procédé selon au moins l'une quelconque des revendications précédentes, **caractérisé en ce que**, après une diminution du bruit dans la fonction de transfert déterminée à partir du rapport entre la statistique réelle et la statistique escomptée avec un signal du patient non altéré ou à partir de la différence de ces fonctions selon une transformation logarithmique, le coefficient d'atténuation et la fréquence de résonance, plus précisément le montant de la fonction de transfert, sont déterminés pour les différentes lignes de fréquence.

27. Procédé selon les revendications précédentes 20 à 26, **caractérisé par** un procédé itératif destiné à améliorer les résultats.

28. Procédé selon au moins l'une quelconque des revendications précédentes, **caractérisé en ce que** la phase est calculée à partir de la fonction de transfert.

29. Procédé selon l'une quelconque des revendications précédentes 1 à 27, **caractérisé en ce que** la phase est déterminée à partir d'une statistique supplémentaire de la forme spécifiée dans les revendications 17 et 18.

30. Procédé selon au moins l'une quelconque des revendications précédentes, **caractérisé en ce qu'**à partir de l'amplitude et de la phase de la fonction de transfert ainsi déterminée sont déterminés par calcul les inverses complexes de ladite fonction, qui est utilisée sous forme d'enregistrement de données de correction pour une correction dans le domaine de fréquence, ce qui permet d'effectuer une transformation de Fourier par segments des signaux du patient mesurés.

# F i g . 1

| | |
|---|---|
| | Druckwandler — 3 |
| | A / D — 4 |
| Kalibrations-Datensätze — 6 | Signalanalyse- und -verarbeitungseinheit — 5 |
| | Auswerte- bzw. Ausgabeeinheit — 7 |

# Fig. 2

Ln (Ampl) verfälschte Messwerte

Frequenz [Hz]

ln (Ampl) Tippdrucksensor

Frequenz [Hz]

# Fig. 3

Statistik der verfälschten Messwerte

verfälschte
Meßwerte

Statistik der Tip-Druckkurve

Tipdruck–Meßwerte

Messung

Tip-Druckkurve

# Fig. 4

# Fig. 5

Residuen

# Fig. 6

Residuen

# Fig. 7

Residuen